# EUROPEAN PATENT APPLICATION

(11) **EP 3 576 101 A1**
(43) Date of publication of application: **04.12.2019**
(21) Application number: 19172104.2
(22) Date of filing: 01.05.2019
(51) Int. Cl.: G16H 50/80, G16H 70/60

(54) **A SYSTEM FOR DETERMINING PUBLIC SENTIMENT TOWARDS PATHOGENS**

(30) Priority: 01.06.2018 US 201815996341
(71) Applicant: Metabiota, Inc., San Francisco, CA 94104 (US)
(72) Inventor: OPPENHEIM, Benjamin Aaron, San Francisco, CA 94104 (US); SERHIYENKO, Volodymyr, San Francisco, CA 94104 (US); GUERRERO, Jaclyn, San Francisco, CA 94104 (US); AYSCUE, Patrick, San Francisco, CA 94104 (US); BARTHEL, Sarah Cheeseman, San Francisco, CA 94104 (US); MADHAV, Nita, San Francisco, CA 94104 (US); STEFAN, Cristina, San Francisco, CA 94104 (US)
(74) Representative: McKinnon, Alistair James

(57) **Abstract**

A system for a sentiment index includes an interface and a processor. The interface is configured to receive a request to determine the sentiment index. The processor is configured to determine a morbidity sub-index; determine a mortality sub-index; and determine the sentiment index based at least in part on the morbidity sub-index and the mortality sub-index.

## Description

### BACKGROUND OF THE INVENTION

Infectious disease outbreaks can cause substantial societal and economic disruption and damages, even when their direct public health impacts (e.g. infections and deaths) are limited. Relatively small outbreaks may drive large changes in human behavior-in travel, in consumption, in workplace absenteeism, and in patterns of interpersonal contact-if the pathogen causing the outbreak evokes sufficient fear, anxiety, and behavioral change. These behavioral alterations have significant implications for governments, societies, and for both individual businesses and macroeconomic outcomes. However, to date there has not been a system to measure or calculate sentiment associated with a pathogen.

### BRIEF DESCRIPTION OF THE DRAWINGS

Various embodiments of the invention are disclosed in the following detailed description and the accompanying drawings.
Figure 1 is a diagram illustrating an embodiment of a sentiment index system.
Figure 2 is a flow diagram illustrating an embodiment of a process for a sentiment index system.
Figure 3 is a flow diagram illustrating an embodiment for a process for determining a morbidity sub-index.
Figure 4 is a flow diagram illustrating a process for determining a fear score.
Figures 5A and 5B are graphs illustrating embodiments of symptom fear scores.
Figure 6 is a flow diagram illustrating an embodiment of a process for determining a mortality sub-index.
Figure 7 is a flow diagram illustrating an embodiment of a process for a preventability sub-index.
Figure 8 is a flow diagram illustrating an embodiment of a process for a treatability sub-index.
Figure 9 is a flow diagram illustrating an embodiment of a process for a transmission sub-index.
Figure 10 is a flow diagram illustrating an embodiment of a process for using a survey to determine a fear score associated with a transmission mechanism.
Figure 11 is a flow diagram illustrating an embodiment of a process for a novelty sub-index.
Figure 12 is a flow diagram illustrating an embodiment of a process for using a survey to determine the fear score associated with novelty.
Figure 13 is a flow diagram illustrating an embodiment of a process for a sentiment index.
Figure 14 is a line graph illustrating an embodiment of a sentiment index score and ranking for the United States.
Figure 15 is a line graph illustrating an embodiment of a sentiment index in backtesting data set.
Figure 16 is a flow diagram illustrating an embodiment of a process for back testing.
Figure 17 is a graph illustrating an embodiment of estimated media reporting.
Figure 18 is a table illustrating an embodiment of regressions estimating correlates of media outbreak coverage.
Figure 19 is a graph illustrating an embodiment of a relation between a sentiment index and predicted outbreak reporting intensity across pandemic and non-pandemic events.
Figure 20 is a table illustrating an embodiment of a gap between predicted and actual reporting values comparing the sentiment index versus cumulative cases and deaths.
Figure 21 is a flow diagram illustrating an embodiment of a process for a sentiment index to be used to trigger an action when the sentiment index score crosses a threshold.

### DETAILED DESCRIPTION

The invention can be implemented in numerous ways, including as a process; an apparatus; a system; a composition of matter; a computer program product embodied on a computer readable storage medium; and/or a processor, such as a processor configured to execute instructions stored on and/or provided by a memory coupled to the processor. In this specification, these implementations, or any other form that the invention may take, may be referred to as techniques. In general, the order of the steps of disclosed processes may be altered within the scope of the invention. Unless stated otherwise, a component such as a processor or a memory described as being configured to perform a task may be implemented as a general component that is temporarily configured to perform the task at a given time or a specific component that is manufactured to perform the task. As used herein, the term 'processor' refers to one or more devices, circuits, and/or processing cores configured to process data, such as computer program instructions.

A detailed description of one or more embodiments of the invention is provided below along with accompanying figures that illustrate the principles of the invention. The invention is described in connection with such embodiments, but the invention is not limited to any embodiment. The scope of the invention is limited only by the claims and the invention encompasses numerous alternatives, modifications and equivalents. Numerous specific details are set forth in the following description in order to provide a thorough understanding of the invention. These details are provided for the purpose of example and the invention may be practiced according to the claims without some or all of these specific details. For the purpose of clarity, technical material that is known in the technical fields related to the invention has not been described in detail so that the invention is not unnecessarily obscured.

A system for a sentiment index is disclosed. The system includes an interface and a processor. The interface is configured to receive a request to determine a sentiment index. The processor is configured to determine a morbidity sub-index; determine a mortality sub-index; and determine the sentiment index based at least in part on the morbidity sub-index and the mortality sub-index. In some embodiments, the system includes a memory coupled to the processor and configured to provide instructions to the processor.

The Pathogen Sentiment Index (or Sentiment Index or Index) was designed to provide an objective, replicable, system to quantify public fear and anxiety towards infectious diseases and outbreaks. The Index is designed to enable index-based triggers for business interruption and related insurance products. A quantitative metric for a pathogen's potential to spark fear and behavioral change facilitates modeling, as well as mitigation and response actions. The negative sentiment associated with a pathogen can cause a sharp decrease in the demand for goods and services across a wide range of economic sectors, primarily because of aversive behaviors such as social distancing or decreased risk tolerance and associated behavioral changes in work, travel, and consumption. This could create financial losses for businesses. The Index can be used to inform insurance policy writing decisions to manage this type of risk, and can serve as an automated trigger to initiate an insurance payment when a sufficiently severe disease outbreak occurs. The Index can also be used to define a system to determine which pathogens and outbreaks warrant surveillance and monitoring.

The Index design is informed by scientific research on the drivers of public fear and anxiety towards adverse shocks. It integrates data on multiple disease attributes-including symptomology, mortality, prevention, treatment, transmission, and novelty- to generate a fear score and rank for each pathogen (including viruses, bacteria, and other types of microorganisms). The Index is designed to be rapidly updated to incorporate new pathogens, including novel and emerging diseases, and the calculation of the Index can incorporate new data-for example, the development of a new vaccine or refined estimates of mortality risk.

The sentiment index system has been empirically tested and validated against a dataset measuring media reaction to outbreaks, which includes over 200 outbreaks worldwide. The empirical results show a positive, statistically significant and stable relationship between the calculated Pathogen Sentiment Index and media coverage of infectious disease events, demonstrating that more frightening pathogens generate more intense media coverage. The results also suggest that the fear-inducing attributes of a pathogen are further magnified in the case of pandemic events, causing nonlinear rates of increase in reporting.

The Pathogen Sentiment Index is designed to support a number of additional functions and automated processes, including the triggering of insurance payments, the initiation of surveillance for disease outbreaks, and calculations of staffing needs in various settings by providing an index-based measure of the level of public fear associated with an array of pathogens. Many infectious diseases and outbreak scenarios carry the risk of significant economic disruption and losses due to fear reactions that do not scale linearly with the observed number of cases and deaths. These scenarios include, but are not limited to:
- **Low-mortality pathogens with fear-inducing symptoms:** For example, Zika virus is very rarely related to mortality in infected individuals but has caused intense fear and widespread behavioral changes, due to its potential to cause birth defects when infection occurs during pregnancy;
- **High-mortality, low-case count pathogens:** The 2015 Middle East Respiratory Syndrome coronavirus (MERS-CoV) outbreak in South Korea is a notable example. The outbreak was relatively limited in scale, causing fewer than two hundred cases; however, the high case fatality ratio, as well as the fact that the pathogen was novel to the region, led to widespread media coverage and public anxiety; and
- **Poorly understood pathogens:** In some cases, the emergence of a new pathogen will be covered by the media, but gaps in scientific knowledge on the pathogen, its spread, or epidemiologic features will lead to uncertainty in the public perception of the risk posed by the pathogen - for example, early in the human immunodeficiency virus infection or acquired immune deficiency syndrome (HIV/AIDS) epidemic in the United States, incidence and prevalence measures often suffered from reporting error and systematic negative bias in case counts, the nature of transmission of the disease was unclear, and effective methods for prevention had not been well defined. All of these factors contributed to a high degree of fear during the early stages of this epidemic.

The Pathogen Sentiment Index ranks pathogens according to the absolute intensity of public fear and anxiety that each agent is likely to cause during an outbreak or infectious disease event. Public reaction to infectious diseases can vary significantly by geography, as a function of cultural norms that shape how people interpret disease symptoms and impacts, variation in endemicity and familiarity with specific diseases, the availability of treatment, and other factors. The Index is designed to capture this geographic variation and is stratified at the country level.

The unit of analysis is the pathogen, which is scored on a 0-100 scale for each country. This allows users to compare the relative rankings of pathogens within a country of interest, or to assess how the estimated fear associated with a pathogen might vary between countries. Country scores can also be averaged to compute a composite Index value.

The Index is designed to be flexible and extensible. Pathogen scores can be updated easily to reflect changes in disease attributes or other new information, such as the development of a vaccine. The Index can also be extended to include new pathogens, such as emerging infectious diseases or wholly novel pathogens with potential epidemiologic and economic importance.

The system and method for determining a sentiment index enables improved measurement of disease outbreaks and capacity to respond to them. In particular, a computer system is made more efficient in automatic triggering of actions associated with operation workflows or demand interruptions that are not possible without the sentiment index determination or calculation. In the event that a sentiment index computation determines that a new or ongoing outbreak has a sufficiently high sentiment index score, it automatically triggers disease surveillance using enhanced monitoring. The automated process works as follows: once a new outbreak begins, publically-available, relevant surveillance data and pathogen information (e.g., information about number of cases, deaths, symptomology, etc.) is gathered using an internet-scraping tool. These data are fed into the sentiment index computation. The sentiment index is computed, and in the event that the score is above a defined threshold, it triggers a series of actions: first, an electronic mail alert is sent to the person responsible for monitoring to notify that the sentiment index threshold is met; and second, the automated scraping of surveillance information increases in frequency-for example, rather than collecting the information once a week, it is instead collected twice a week. The surveillance information that is gathered is structured, validated, placed into a database, and published for user viewing more frequently as well. The sentiment index score is also dynamically recalculated as new data become available. With each new calculation of the sentiment index score, the score is assessed against the predefined threshold to see if the more frequent monitoring continues to be necessary, or if less frequent monitoring would be adequate.

in some embodiments, another capability is made possible by the Sentiment Index - to gauge the level of fear and interest that a pathogen will generate, which can be used to forecast the number of inquiries that would be received about a pathogen during an ongoing outbreak, which can help to determine the number of resources (e.g., call center employees) that would need to be available to efficiently handle that volume of interest during a crisis. The automated process works as follows: once a new outbreak begins, publicly-available, relevant surveillance data and pathogen information (e.g., information about number of cases, deaths, symptomology, etc.) is gathered using an internet-scraping tool; second, these data are fed into the sentiment index computation; third, the sentiment index is computed, and in the event that the score is above a defined threshold, it triggers a system that first calculates the expected number of additional call center staff (e.g., the additional staff is a function of the sentiment index score - for example, a linear function, a non-linear function, etc.), and second, initiates a series of automated phone calls to a predefined list of potential call center workers who serve as "surge capacity". During the automated phone calls, the potential workers who are available to join the call center would press a button on their phone to indicate their acceptance of the assignment. Once the required number of workers have accepted the assignment, the automated phone calls would cease. The workers would then report to the call center to handle the increased call volume and continue to staff the center until the crisis has subsided.

In some embodiments, the Sentiment Index is directly linked to automated processes such as inventory management systems, in order to dynamically adjust orders due to estimated changes in public behavior and demand.

In some embodiments, the system is deployed in a range of industries and sectors where consumer demand is likely to be impacted by fear of contact and exposure to disease, including consumer packaged goods, restaurants and hospitality, among others. Following the initial detection of an epidemic event, a Sentiment Index score is computed. The severity of this score provides a predictive estimate of potential consumption changes, which are used as a parameter to drive adjustments in stock volume and ordering. This kind of mechanism ensures the efficiency of supply chain systems is maintained and the disruptions are minimal, with an equilibrium being reached at a new intersection point between demand and supply curves.

In the example of a restaurant business that is ordering the necessary food supplies for a multitude of producers, the system is as follows:
- an indication is received that an epidemic outbreak has started (i.e., first case/death is observed);
- sentiment index scoring is initiated, and an index value is computed;
- a public fear value is determined based at least in part on the index value and provided to related entities (e.g., a related entity to the restaurant business are suppliers and producers)
- Once the sentiment index has reached a certain critical threshold, then the restaurant acquisition team is notified and the ordering system automatically adjusts daily the ordering volumes of food supplies.
The producers benefit from the early detection mechanism supported by the Sentiment Index since they plan to re-route their excess stocks to other markets that are not yet impacted by the outbreak. In this way, both the supply and demand parties have early warning about potential disruptions to their normal, expected business volumes. The process results in minimal supply chain disruptions and lower economic loss.

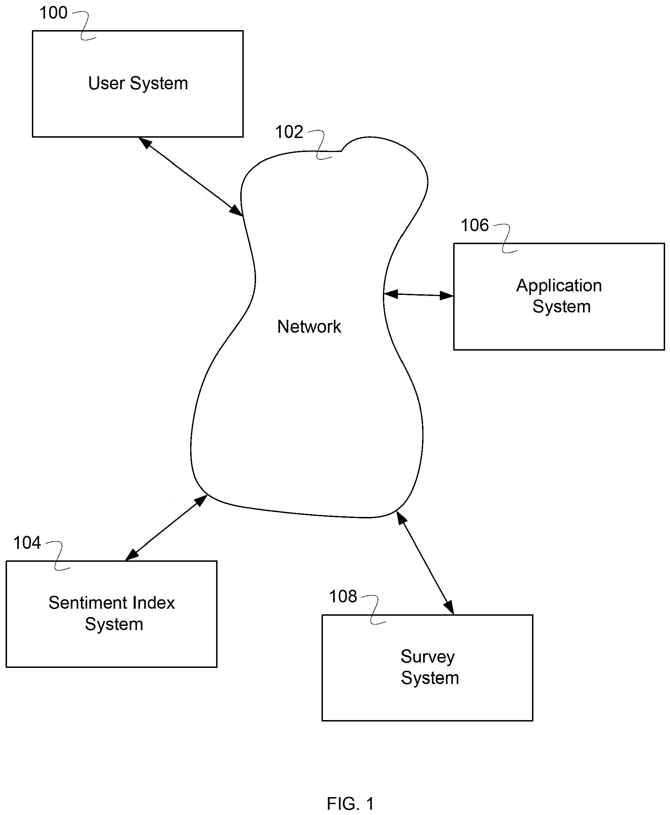

Figure 1 is a diagram illustrating an embodiment of a sentiment index system. In the example shown, a user using user system 100 requests a sentiment index. Sentiment index system 104 receives the request via network 102 and determines the sentiment index. The sentiment index system 104 includes an interface and a processor. The interface is configured to receive a request to determine the sentiment index. The processor is configured to determine a morbidity sub-index; determine a mortality sub-index; determine a preventability sub-index; determine a treatability sub-index; determine a transmission sub-index; determine a novelty sub-index; and determine the sentiment index based at least in part on the morbidity sub-index, the mortality sub-index, the preventability sub-index, the treatability sub-index, the transmission sub-index, and the novelty sub-index. Some of the sub-indices use survey system 108 to determine the sub-indices. For example, sentiment index system 104 requests survey results from survey system 108 via network 102 and receives survey results from survey system 108 via network 102. In some cases the value of the sentiment index is provided as an indication to application system 106 to trigger an action, payment, or decision.

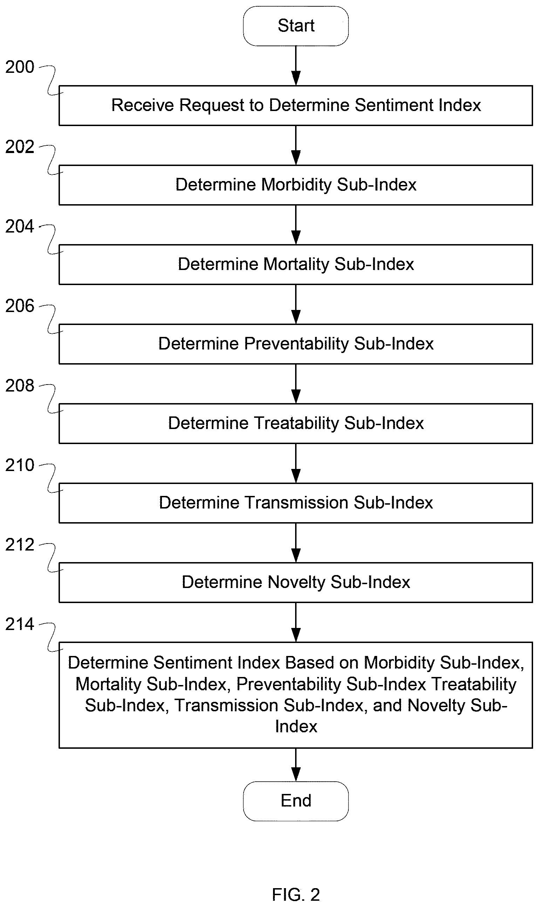

Figure 2 is a flow diagram illustrating an embodiment of a process for a sentiment index system. In some embodiments, the process of Figure 2 is implemented by a sentiment index system (e.g., sentiment index system 104 of Figure 1). In the example shown, public fear and anxiety regarding infectious diseases is considered a function of a number of factors:
- **Morbidity**: the set of symptoms commonly caused by infection with each pathogen;
- **Mortality**: the risk of death associated with infection;
- **Prevention:** whether the disease can be prevented prophylactically through vaccination or chemoprophylaxis;
- **Treatment**: whether the disease can be cured or ameliorated by a therapeutic course;
- **Transmission mechanism**: how the disease is typically contracted or spread in an epidemic setting; and
- **Novelty**: how new, unknown, or unusual the pathogen is.
Each factor is measured via a sub-index. Pathogens are scored by taking a weighted combination of a set of the six sub-indices (e.g., one, two, three, four, five, or six of the sub-indices), with weights reflecting the relative importance of each component. The Sentiment Index is designed to capture variation between countries to the greatest extent possible. Several sub-indices are computed from nationally-representative population survey data and therefore vary by country. Similarly, sub-index weights vary by country, reflecting the varying importance that national populations place on discrete attributes of infectious diseases . Other sub-indices are measured via administrative or other observational data, which does not vary by country. Sub-indices vary by geography (e.g., morbidity, transmission, novelty, and sentiment index weights vary by country; mortality, prevention, and treatment sub-indices are global). In some embodiments, additional sub-indices are also used in the calculation of the Sentiment Index.

In 200 a request is received to determine the sentiment index. In 202, a morbidity sub-index is determined. In 204, a mortality sub-index is determined. In 206, a preventability sub-index is determined. In 208, a treatability sub-index is determined. In 210, a transmission sub-index is determined. In 212, a novelty sub-index is determined. In 214, the sentiment index is determined based at least in part on the morbidity sub-index, the mortality sub-index, the preventability sub-index, the treatability sub-index, the transmission sub-index, and the novelty sub-index.

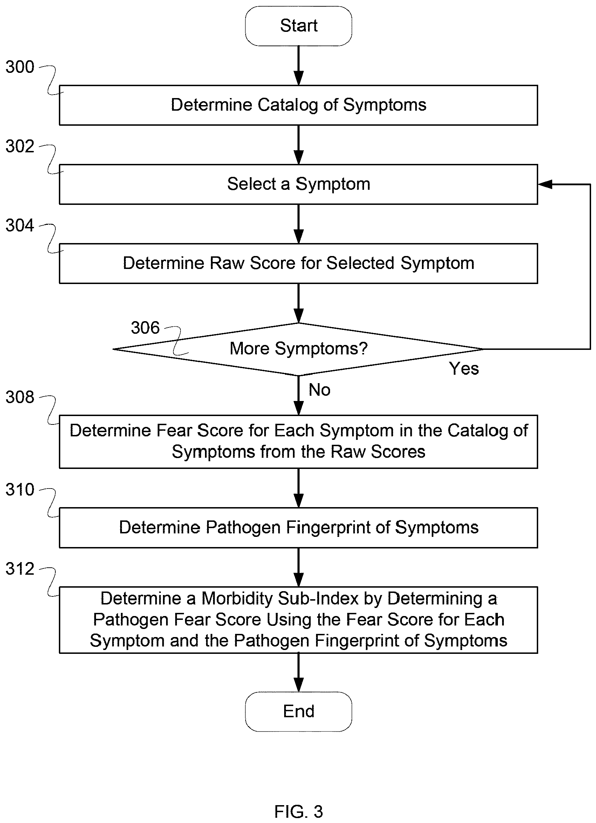

Figure 3 is a flow diagram illustrating an embodiment for a process for determining a morbidity sub-index. In some embodiments, the process of Figure 3 is used to implement 202 of Figure 2. In the example shown, clinical presentation of a disease - the typical set of symptoms caused by the pathogen - is an important driver of individual fear and anxiety. Common symptoms (also described as morbidities) become associated with a pathogen through media coverage and the diffusion of information across social networks. In general, the public is likely to be more fearful of severe, grotesque, painful, unpleasant, and longer-duration (or permanent) symptoms. Diseases with more severe morbidities also receive greater media coverage, which can further amplify public knowledge and anxiety about a specific pathogen. The Morbidity sub-index measures public fear and aversion towards the symptoms associated with infection by each pathogen covered in the Index. A specific symptomatic profile is constructed for each pathogen and then matched against a symptom catalog that uses cross-national survey data to measure public fear of distinct disease signs and symptoms.

Symptom profiles were constructed for each pathogen through a cross-validation of multiple data sources, including public and privately held databases, for example published scientific research on infectious diseases and their attributes, clinical decision support guidelines, and pathogen profiles maintained by the US Centers for Disease Control and the World Health Organization (WHO).

Symptoms vary in terms of the frequency of their clinical presentation. Some symptoms are relatively rare, and only present in a subset of cases; for example, encephalitis associated with measles infection is relatively unusual. A strictly rational and evidence-based estimation of risk would take the rarity of symptoms into account when considering the morbidity caused by a specific pathogen. However, media reports often note the most severe or sensational symptoms, without taking symptom frequency into account. For example, media reporting on measles outbreaks and risk, has highlighted relatively rare and serious consequences of infection, such as encephalitis, without indication of frequency of occurrence. The Sentiment Index design takes this into account by weighting severe symptoms more heavily (i.e., by assigning a higher score).

Data are pre-processed prior to constructing the symptom profiles. Duplicates are removed, to prevent "double-counting" highly similar symptoms which would be functionally indistinguishable to a patient. For example, bronchiolitis and bronchitis can be considered as a group. Second, for pathogens which can lead to congenital birth defects, parent and child-related symptoms are merged. For example, the symptom profile for Zika contains both microcephaly-the most drastic and observable potential impact of infection during a pregnancy--as well as the cold and flu-like symptoms experienced by an infected parent.

The Symptom catalog was constructed by extracting all symptoms from a large universe of pathogens, as well as symptoms contained within clinical decision support tools and diagnostic guidelines.

Population surveys were administered in individual countries. Surveys may be administered face-to-face, by phone (including interview, **SMS** or interactive voice response systems, or online via web-based systems). The surveys are weighted by age and gender to construct nationally representative samples. Respondents are asked a range of questions, including a battery of questions designed to measure the relative public fear and aversion directed towards each symptom. The survey question presents each respondent with a randomly-drawn set of symptoms, and asks the respondent to pick the symptom that they found most frightening or distressing. This process is repeated iteratively. The weighted sum of these "votes" (i.e., symptom selections from within each randomly-generated grouping) is tabulated to provide an overall score for each symptom. The symptoms are then ranked, and assigned a score based on their rank within the catalog. In various embodiments, symptoms are ranked and then divided into quintiles, deciles, or any other appropriate strata, in order to simplify scoring. In some embodiments, "raw" rank scores are used to compute the Morbidity sub-index.

A scoring algorithm then links each pathogen's symptomatic profile with the catalog, to estimate an overall morbidity score. The algorithm is designed to weight towards the most fear-inducing symptoms, again reflecting the fact that these symptoms are likely to receive the most media coverage and so become closely associated with the pathogen. For example, although infection with *Zaire ebolavirus* can cause headaches, more severe symptoms such as hemorrhaging are most strongly associated with the pathogen.

In some embodiments, the algorithm sums the three highest scoring (i.e., the most frightening) symptoms from the symptom profile, and then adds the weighted sum of the remaining symptoms. In some embodiments, the weighted sum is computed by summing the remaining symptoms, and multiplying by a weight (e.g., a weight of 1/9); the weight is derived by taking the maximum number of symptoms that can be assigned to a pathogen (for example, 9, 12, 14, etc.), minus the three most severe symptoms. In some embodiments, other numbers of severe symptoms can be selected. Most generally, the algorithm sums the k highest symptoms (i.e., the most frightening) scores symptoms from the symptom profile, and then adds the weighted sum of the remaining symptoms. The weighted sum is computed by summing the remaining symptoms, and multiplying by the weight; the weight is derived by taking the maximum number of symptoms (e.g., m) that can be assigned to a pathogen, minus the k most severe symptoms (i.e., m-k).

In 300, a catalog of symptoms is determined. In 302, a symptom is selected. In 304, a raw score is determined for the selected a symptom. In 306, it is determined whether there are more symptoms. In the event that there are more symptoms, control passes to 302. In the event that there are no more symptoms, in 308 determine a fear score for each symptom in the catalog of symptoms from the raw scores. In 310, a pathogen fingerprint of symptoms is determined. For example, a pathogen fingerprint comprises a set of symptoms that characterizes the pathogen and its effects. In 312, a morbidity sub-index is determined by determining a pathogen fear score using the fear score for each symptom and the pathogen fingerprint of symptoms.

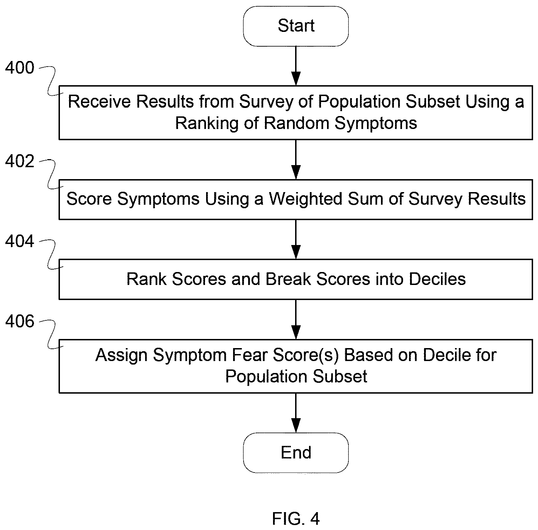

Figure 4 is a flow diagram illustrating a process for determining a fear score. In some embodiments, the process of Figure 4 is used to implement 308 in Figure 3. In the example shown, in 400 results are received from a survey of a population subset using a ranking of symptoms. For example, survey results are received from a survey system (e.g., survey system 108 of Figure 1). In 402, symptoms are scored using a weighted sum of survey results. In 404, scores are ranked and the scores are broken into deciles. In 406, the symptom fear score(s) is/are assigned based on the decile for the population subset. In various embodiments, fear scores are derived from raw (i.e., total) votes, percentiles, stack rank, k-means clustering, or any other appropriate sorting and/or ranking processes.

In some embodiments, the Morbidity sub-index can be calculated for specific populations of interest - for example, by gender, or within particular age bands, or for other sub-populations.

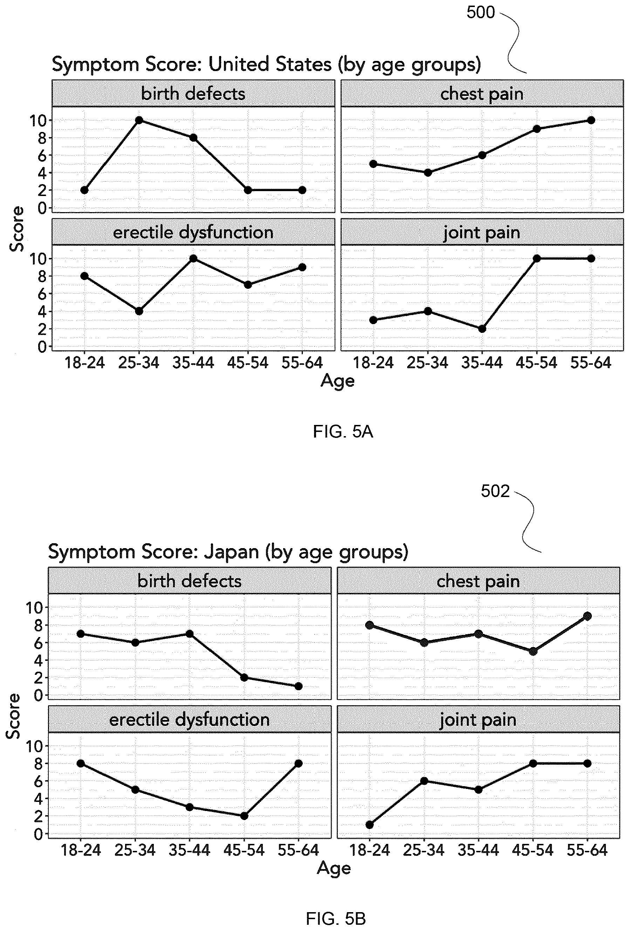

Figures 5A and 5B are graphs illustrating embodiments of symptom fear scores. In some embodiments, the results graphed in Figures 5A and 5B are generated using the process of Figure 4. In the examples shown, graphs in Figure 5A show fear of the symptoms of birth defects, chest pain, erectile dysfunction, and joint pain as a function of age for survey respondents in the United States. Graphs in Figure 5B shows fear of the symptoms of birth defects, chest pain, erectile dysfunction, and joint pain as a function of age for survey respondents in Japan. These graphs illustrate variation in symptom scores by age and gender - for example, greater fear of birth defects and sexual dysfunction among people within reproductive age bands. As indicated by the figures, this variation can be significant. Sub-population specific Morbidity scores can be used in a variety of applications, including triggers for insurance policies that are tailored to the needs of the specific end-user: for example, for a hotel that caters to young tourists and newlyweds, an Index score for these age bands may be computed to ensure that outbreaks of concern to this particular clientele would trigger a payout under appropriate conditions.

The surveys used to determine fear scores underwent multiple rounds of pilot testing, in order to test and refine question wording, response structures - for example, Likert-scale responses for symptoms, versus the "voting" structure employed in the final survey - and item and battery sequencing. Multiple indicators, including item non-response, overall respondent attrition levels, and attrition rates across gender and age bands were considered. The final survey questionnaire was translated by professional translators, back-translated to ensure proper translation of all items (particularly complex terms such as disease symptomologies and morbidities). The survey design included randomized item ordering, to mitigate potential bias introduced by sequencing effects.

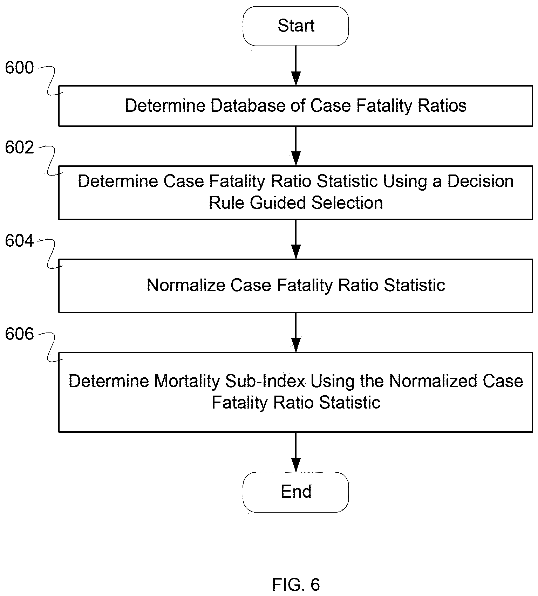

Figure 6 is a flow diagram illustrating an embodiment of a process for determining a mortality sub-index. In some embodiments, the process of Figure 6 is used to implement 204 of Figure 2. In the example shown, research on risk perception shows a generally positive correlation between public anxiety and risk severity: risks with fatal consequences are viewed with greater fear than non-fatal risks. The mortality risk associated with infection is typically expressed in simple terms - the case-fatality ratio (CFR), the estimated proportion of cases who die from the disease - that allows for easy interpretation by the public. In general, greater mortality risk should be positively correlated with fear.

Mortality risk is measured via CFR. CFR estimates were drawn from statistics from the World Health Organization (WHO), US Centers for Disease Control, the European Centre for Disease Prevention and Control, the Public Health Agency of Canada, peer-reviewed publications, estimates provided by Ministries of Health, and non-governmental public health organizations.

CFR estimates vary widely according to the level of detail available and the types of assumptions that are made. If multiple CFR estimates were identified, a decision-rule guided the selection of a single CFR statistic, with the CFR reported for cases receiving standard therapies in developed-country settings given preference. If multiple clinical manifestations can result from infection with the same pathogen (including strain/serotype), CFR was aggregated across the clinical spectrum in order to capture the average estimated mortality rate arising from infection.

A CFR statistic is taken using the decision rule described above. For many pathogens, the scientific literature and public health agencies may report a wide range of CFR estimates. In one embodiment of the Index, where the CFR is estimated as a range (e.g. 5-8%), the highest value of the range was selected, reflecting the assumption that the public will likely focus upon the high-end estimate and the most severe potential consequence of infection. The resulting statistic naturally falls between 0 (no deaths), and 100 (all cases estimated to result in death), and is recomputed or normalized to range from 0-1.

In 600, a database is determined of case-fatality ratios. In 602, case-fatality ratio statistic is determined using a decision rule guided selection. In 604, the case-fatality ratio statistic is normalized. In 606, a mortality sub-index is determined using the normalized case-fatality ratio statistic.

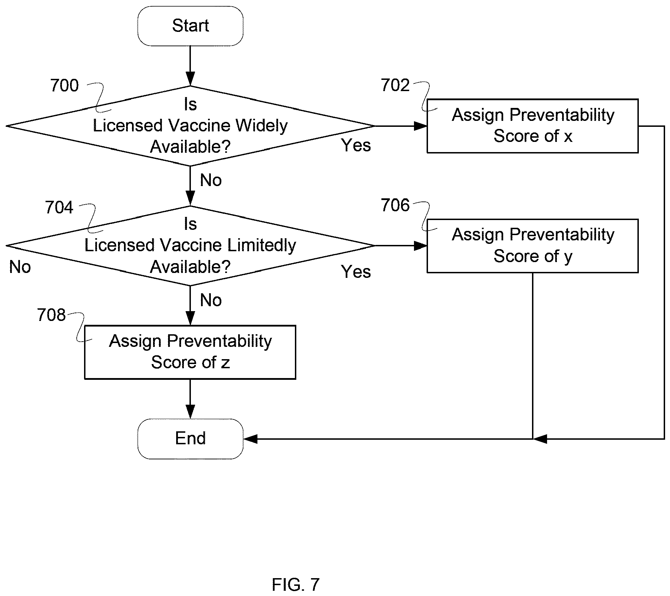

Figure 7 is a flow diagram illustrating an embodiment of a process for a preventability sub-index. In some embodiments, the process for Figure 7 is used to implement 206 of Figure 2. In the example shown, empirical research on risk perception has shown that risks which cannot be prevented or mitigated are viewed with much higher levels of dread. The availability of vaccines or other prophylaxis is likely to reduce fear, and the availability of therapeutics (even with limited efficacy) and should also reduce fear, by providing some feeling of agency and control in the case of infection.

Information on vaccine availability and therapeutic options were collected from similar sources as the CFR data: from the World Health Organization (WHO), the US Centers for Disease Control, the European Centre for Disease Prevention and Control, and the Public Health Agency of Canada, supplemented as required by peer-reviewed scholarly publications.

Each pathogen was categorized regarding the availability of prophylactic vaccine, as follows:
- **Yes**: A licensed vaccine is widely available (or perceived as widely available by the public;
- **Limited**: A vaccine exists but is not perceived to be widely available - for example, vaccine may be provided for high risk personnel only (i.e., military, laboratory workers, veterinarians), for emergency investigational use during outbreaks, or the source may state overtly that there is no vaccine widely available; and
- **No**: Licensed vaccine is unavailable; no vaccine is readily available for outbreak settings or high risk populations.

A pathogen without a vaccine is assigned a high score of z (e.g., 1), a vaccine with limited availability is assigned a medium score of y (e.g., 0.25), and a vaccine with wide availability is assigned a low score of x (e.g., 0). The score for limited availability reflects the fact that potential shortages and inability to access resources needed to limit risk is known to generate fear and anxiety, primarily owing to the uncertainty and doubt that become associated with the potential mechanism to avoid harm.

In 700 it is determined whether a licensed vaccine is widely available. In the event that a licensed vaccine is widely available, then in 702 a preventability score of x is assigned, and the process ends. For example, a high score is assigned (i.e., 1). In the event that a licensed vaccine is not widely available, then in 704, it is determined whether a licensed vaccine is limitedly available. In the event that a licensed vaccine is with limited availability, then in 706 a preventability score of y is assigned, and the process ends. For example, a medium score is assigned (i.e., 0.25). In the event that a licensed vaccine is not limitedly available, then in 708 a preventability score of z is assigned, and the process ends. For example, a low score is assigned (i.e., 0).

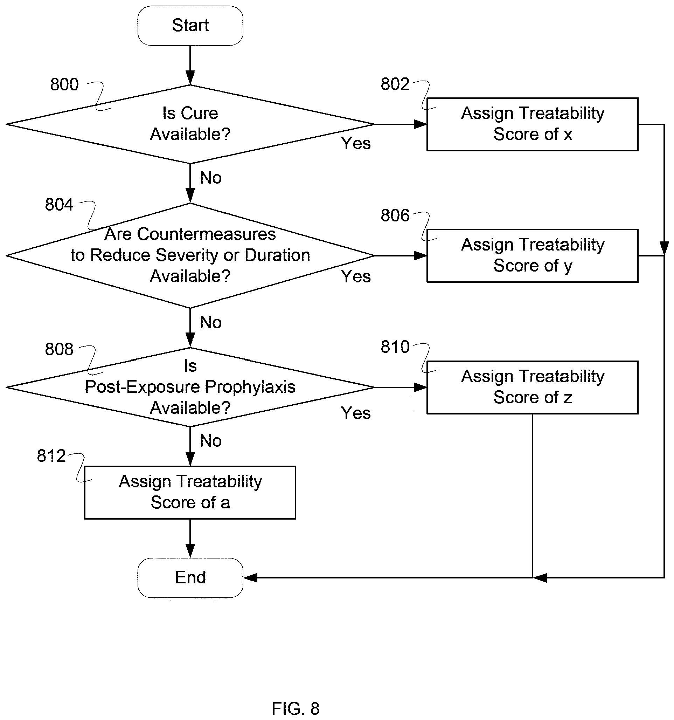

Figure 8 is a flow diagram illustrating an embodiment of a process for a treatability sub-index. In some embodiments, the process for Figure 8 is used to implement 208 of Figure 2. In the example shown, each pathogen was categorized regarding the availability of specific therapeutic interventions, as follows:
- **No**: No therapeutic intervention exists that is known to cure, reduce the severity or duration of illness, or prevent disease or improve outcome;
- **Post-exposure prophylaxis only**: No therapeutic intervention exists that is known to cure or reduce the severity/duration of illness, but options exist to administer effective post-exposure prophylaxis to prevent disease or improve outcome after exposure to a pathogen
- **Therapeutic**: Therapeutic interventions exist that may reduce the severity or duration of illness:
   ∘ Licensed or recommended therapy specifically for the target pathogen; and
   ∘ Not licensed for the target pathogen, but published evidence and clinical guidance demonstrates that it can be used to treat cases of the specific pathogen with success or benefit
- **Curative**: Therapeutic interventions exist that can cure the disease.

A pathogen without any therapeutic options is assigned a high value score of a, post-exposure prophylaxis only, a medium high value score of z; therapeutic, a medium value score of y; and curative, a low value score of x. The score progression reflects the fact that post-exposure prophylaxis is administered following potential exposure and may not be completely effective, thereby posing a frightening scenario for a potentially infected person. The availability of a therapeutic option is assigned a score at the midpoint of the scale, since the effects of infection can be ameliorated but not completely cured. Curative is assigned a score of x, since the patient may perceive that they can (upon treatment) completely resume life after infection.

In 800 it is determined whether a cure is available. In the event that a cure is available, then in 802 a treatability score of x is assigned, and the process ends. For example, a low value (i.e., 0). In the event that a cure is not widely available, then in 804, it is determined whether countermeasures to reduce severity or duration are available. In the event that countermeasures to reduce severity or duration are available, then in 806 a treatability score of y is assigned, and the process ends. For example, a medium value (i.e., 0.5). In the event that countermeasures to reduce severity or duration are not available, then in 808 it is determined whether post-exposure prophylaxis is available. In the event that post-exposure prophylaxis is available, then in 810 a treatability score of z is assigned, for example, a medium high value (i.e., 0.75), and the process ends. In the event that post-exposure prophylaxis is not available, then in 812 a treatability score of a is assigned, for example, a high value (i.e., 1), and the process ends.

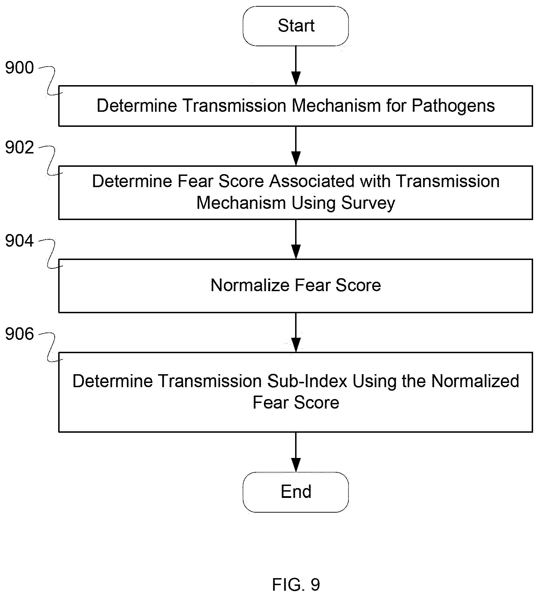

Figure 9 is a flow diagram illustrating an embodiment of a process for a transmission sub-index. In some embodiments, the process of Figure 9 is used to implement 210 of Figure 2. In the example shown, the fear associated with a pathogen is also shaped by the perceived risk of acquiring an infection. This assessment is highly subjective, and often not correlated with the underlying probability of infection. During an outbreak, the public will search for cues regarding the risk of infection. These may include localized incidence and prevalence rates, but for the majority of the public, simpler heuristics like the mechanism of transmission may be more informative and interpretable.

Data on the transmission mechanism for each pathogen was drawn from scientific and public health literature, including published scientific papers, as well as pathogen profiles maintained by the US Centers for Disease Control, the World Health Organization (WHO), the Public Health Agency of Canada, and other public health agencies. The mechanism that is most commonly associated with transmission in the context of epidemics was selected, as opposed to uncommon rare but theoretically possible mechanisms or those that frequently differ in the context of endemic and epidemic events. In cases where a pathogen can be spread through multiple transmission mechanisms-where a single mechanism could not be determined based upon scientific literature-the most fear-inducing mechanism was selected, based upon survey results. This decision criterion is based on the assumption that the most fear-inducing mechanism will also be the most commonly reported by the news media and will also be the most widely recalled by members of the public exposed to media reporting on an outbreak event.

The fear score associated with each disease transmission mechanism is derived from population survey data. In one embodiment, disease transmission fear scores are estimated through a survey vignette technique. In this embodiment, each respondent is randomly selected to answer a different question about a hypothetical disease outbreak. Every question (and hypothetical outbreak scenario) is identical, except for the pathogen's mechanism of transmission. Respondents are asked to rate their degree of fear regarding the scenario on a Likert scale (range: 1-5), and differences in average (mean) fear levels across the scenarios is used to estimate the relative fear associated with each type of disease transmission. The mechanism with the highest fear score is assigned a value of 1, and other mechanisms are scored proportionally. The proportion may be estimated linearly - for example, if airborne is rated at 4.0, and bloodborne at 3.5, airborne would be scored as 1, and bloodborne as 0.875 - logarithmically, or via other techniques. In other embodiments, other survey question techniques may be used, such as direct elicitation of fear regarding transmission mechanisms, with respondents indicating their level of fear via a Likert scale or other ordinal, interval, or ratio-level response variable.

In 900, a transmission mechanism for pathogens is determined. For example, transmission mechanisms could include airborne transmission, direct transmission, bloodborne transmission, food transmission, water transmission, sexual transmission, or any other appropriate transmission mechanism. In 902, a fear score associated with the transmission mechanisms is determined using a survey. In 904, the fear score is normalized. In 906, a transmission sub-index is determined using the normalized fear score.

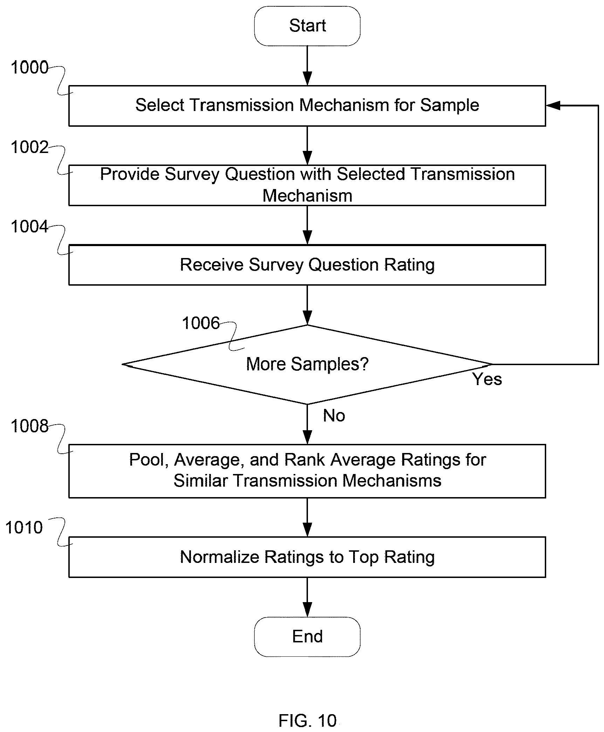

Figure 10 is a flow diagram illustrating an embodiment of a process for using a survey to determine a fear score associated with a transmission mechanism. In some embodiments, the process of Figure 10 is used to implement 902 of Figure 9. In the example shown, in 1000 a transmission mechanism is selected for a sample. In 1002, a survey question is provided with the selected transmission mechanism. In 1004, a rating is received for the survey question. In 1006, it is determined whether there are more samples. In the event that there are more samples, control passes to 1000. In the event that there are not more samples, in 1008 ratings are pooled, averaged, and ranked for similar transmission mechanisms. In 1010, ratings are normalized to top ratings.

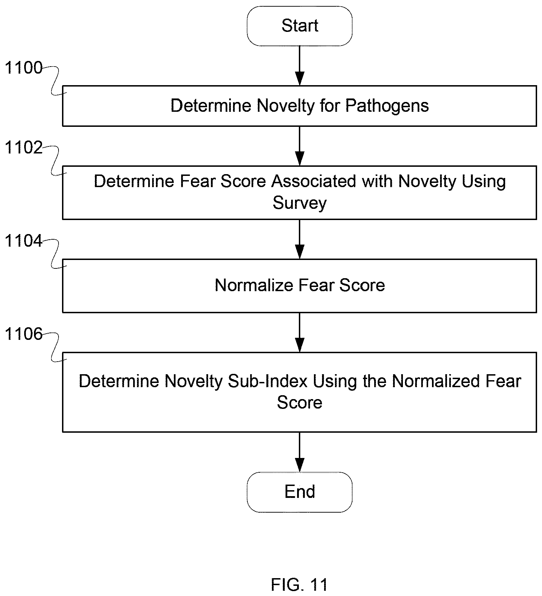

Figure 11 is a flow diagram illustrating an embodiment of a process for a novelty sub-index. In some embodiments, the process of Figure 11 is used to implement 212 of Figure 2. In the example shown, research on risk perception has found that unfamiliarity amplifies the perception of risk. Precisely why is not clear, but the absence of information may lead people to make worst-case assumptions in order to adopt a conservative (and safe) posture towards potentially severe risk. Accordingly, a novel or emerging pathogen is likely to cause more fear than the outbreak of a known or endemic disease.

In some embodiments, pathogen novelty is coded at the country level and created by taking into account the rarity of outbreaks. Extremely rare pathogens are likely to be considered unusual and attract attention and anxiety, even in countries where scattered cases or outbreaks have occurred. In some embodiments, a pathogen is coded as novel if a country has not experienced outbreaks or locally arising cases, beneath a threshold - for example, 50 cases. This approach allows for the appropriate scoring of pathogens that may be established (or emerging) into human populations in some regions, but have not yet reached others. For example, *Zaire ebolavirus* is not considered novel in the Democratic Republic of the Congo, where the disease reservoir is present and local outbreaks have occurred, but is considered novel in the United States. Relatedly, a pathogen is still coded as novel if it has caused infections solely from an imported case or within a laboratory setting, but has not become established in the local population. In some embodiments, pathogen novelty is scored at the regional level, or at other levels of geographic resolution. Novelty may also be coded based upon other criteria besides deaths, including the number of outbreaks over a period of time (for example, the preceding decade), the level of scientific knowledge regarding a particular pathogen, or whether there has been a recent change in scientific understanding of a pathogen (for example, the determination that Zika virus could cause microcephaly) that could rapidly alter the public's fear towards a pathogen.

The fear score associated with novelty is derived from the population survey. In some embodiments, each respondent is randomly assigned to one of three survey questions describing a hypothetical outbreak: a "baseline" scenario, a novel outbreak version, and a drug-resistant version. The three versions are identical in terms of cases, scale, and progression, varying only according to whether the pathogen is additionally described as either novel (newly emerging, not understood by scientists), or known and resistant to drugs and therapies. The difference in survey responses between baseline, novel, and resistant versions is used to estimate the additional level of fear generated by these pathogen features. The scenario with the highest fear score is assigned a value of 1, and other versions are scored proportionally. In some embodiments, other survey question techniques may be used, including direct questions regarding respondent fear levels as measured by a Likert scale or other ordinal, interval, or ratio-level response variable.

In 1100 a novelty score is determined for pathogens. In 1102, a fear score is determined associated with novelty using a survey. In 1104, the fear score is normalized. In 1106, a novelty sub-index is determined using the normalized fear score.

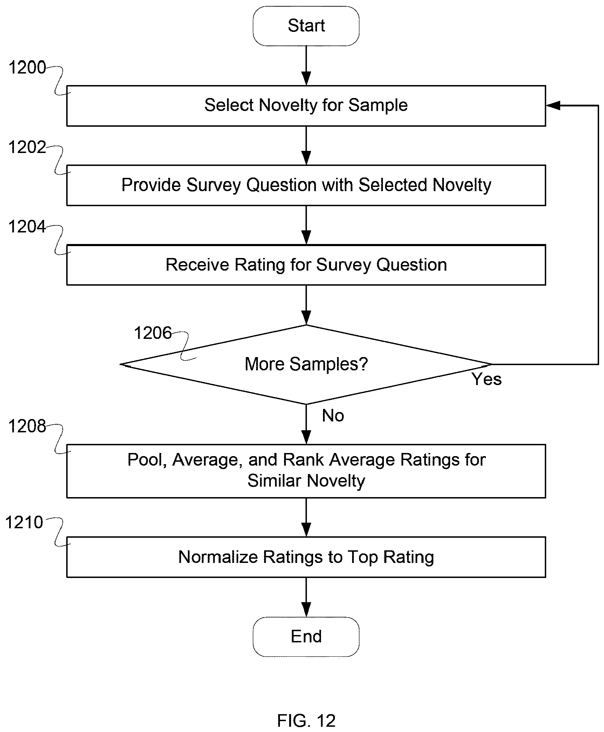

Figure 12 is a flow diagram illustrating an embodiment of a process for using a survey to determine the fear score associated with novelty. In some embodiments, the process of Figure 12 is used to implement 1102 of Figure 11. In the example shown, in 1200 a novelty is selected for a sample. In 1202, a survey question is provided with the selected novelty. In 1204, a rating is received for the question. In 1206, it is determined whether there are more samples. In the event that there are more samples, control passes to 1200. In the event that there are not more samples, in 1208 ratings are pooled, averaged, and ranked for similar novelty. In 1210, ratings are normalized to top ratings.

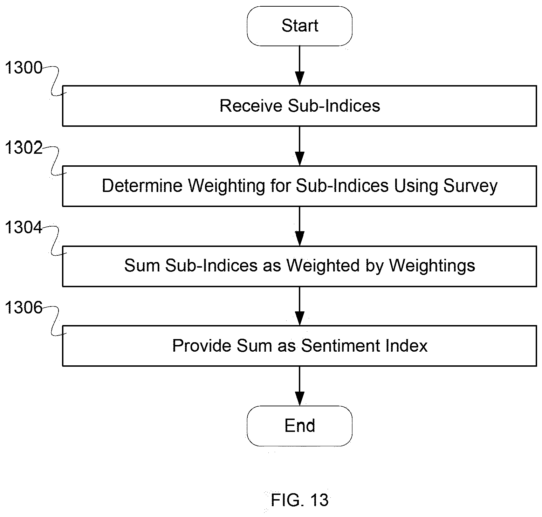

Figure 13 is a flow diagram illustrating an embodiment of a process for a sentiment index. In some embodiments, the process of Figure 13 is used to implement 214 of Figure 2. In the example shown, the underlying data for each sub-index are scaled so that all indices have equal "natural" weight, ranging from 0-1, before being modified by design weights.

Design weights are calculated directly from the population surveys, using a survey question that measures the relative importance of each sub-index. In one embodiment, respondents were presented with a hypothetical disease outbreak in a city that they were about to visit, and asked to identify which consideration would be most important in influencing their likelihood of cancelling (or completing) their trip. Other estimation techniques, including direct questions regarding the importance of each factor as a driver or fear or behavioral change, may be utilized, and measured by a Likert scale or other ordinal, interval, or ratio-level response variable.

The weights are computed by estimating the proportion of respondents that select each factor (e.g., morbidity, mortality, transmission, prevention, treatment, novelty, etc.). For example, if 25 percent of the sample selected vaccine availability as the most important factor, the weight for vaccine would be 0.25. The weights vary by country, reflecting the differing importance that each country population places on each dimension of risk.

In 1300, sub-indices are received. In 1302, weighting is determined for sub-indices using a survey. In 1304, sub-indices are summed as weighted by weightings. In 1306, sum is provided as the Sentiment Index.

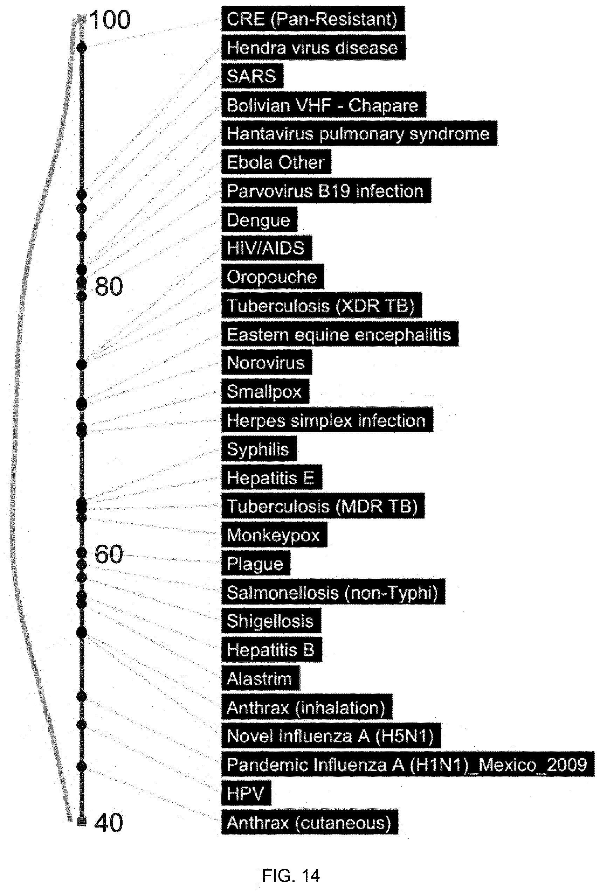

Figure 14 is a line graph illustrating an embodiment of a sentiment index score and ranking for the United States. In the example shown, Index scores are computed by taking the weighted sum of all index dimensions. In this embodiment, the Index score is absolute, rather than relative: the scores are not normalized, in order to demonstrate the potential space for new (unranked or unknown) pathogens which could be subsequently added to the catalog. However, in some embodiments the Index scores are normalized and recomputed as a relative ranking.

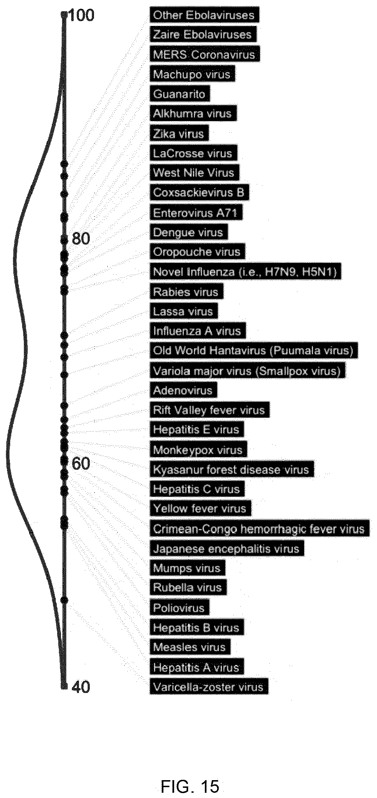

Figure 15 is a line graph illustrating an embodiment of a sentiment index in backtesting data set. In the example shown, in order to validate the Pathogen Sentiment Index and explore its predictive properties, an empirical analysis was conducted to examine the association between Index scores and historical patterns of media reporting during a wide range of outbreak events. The analysis used the United States Index scores to model English-language reporting.

The backtesting process consisted of three steps: the creation of a historical outbreak dataset, the extraction of data on news media reporting for sampled outbreak events, and the construction of statistical models and robustness checks. These steps are described below, followed by the results of the backtesting.

The outbreak dataset was designed to cover a ten year window, from 2007- 2017. This window was selected because it follows the passage (and global monitoring and enforcement) of the International Health Regulations, which introduced greater rigor and uniformity in global outbreak reporting. Events were sampled from peer-reviewed scientific literature, in order to capture smaller, geographically contained events which would not necessarily result in a World Health Organization Disease Outbreak News (WHO DON) or other international report. Data on cumulative cases and deaths were extracted for each event from peer reviewed articles and/or ProMED. A target of 10 outbreaks per pathogen was set, in order to explore variation in reporting within specific pathogens, and to mitigate the potential for haphazard variation in the news cycle around specific outbreak events to bias estimates. Endemic diseases were sampled if they are known to sporadically transition to epidemic phases; for example, dengue and yellow fever are endemic to a wide range of countries, but also known to reach epidemic levels.

Lastly, a case threshold was applied to historical events: events were sampled if they resulted in at least 100 cases. This threshold was applied in part to screen out disease emergence events, where contemporaneous media reporting would not necessarily include a consistent pathogen name, and thus bias estimates of media coverage. The 100 case threshold would also not trigger a payout on a hypothetical policy with a 50 death trigger except for exceptionally deadly pathogens (CFR >=0.5), but would still constitute a large enough event to potentially attract regional and global media attention.

The outbreak dataset includes 221 outbreak events caused by 35 pathogens. Figure 15 shows the distribution of Sentiment Index scores for the pathogens included in the backtesting dataset. The backtesting dataset includes pathogens across the full range of index, from low scoring, innocuous agents, to severe and high-ranked pathogens.

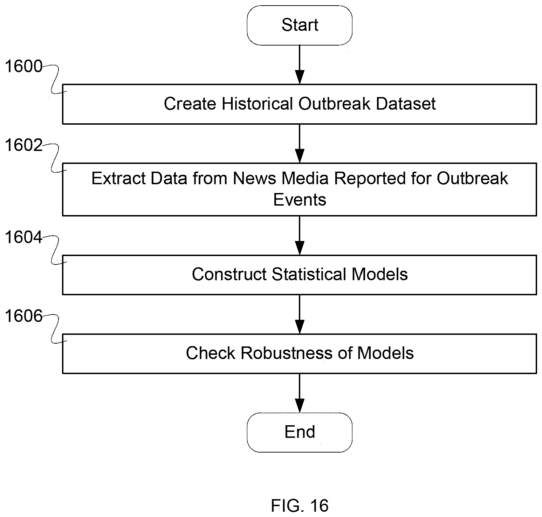

Figure 16 is a flow diagram illustrating an embodiment of a process for back testing. In some embodiments, the process of Figure 16 is used with the backtesting data of Figure 15. In the example shown, in 1600 a historical outbreak dataset is created. In 1602, data are extracted from news media reported for outbreak events. In 1604, statistical models are constructed. In 1606, robustness of models is checked.

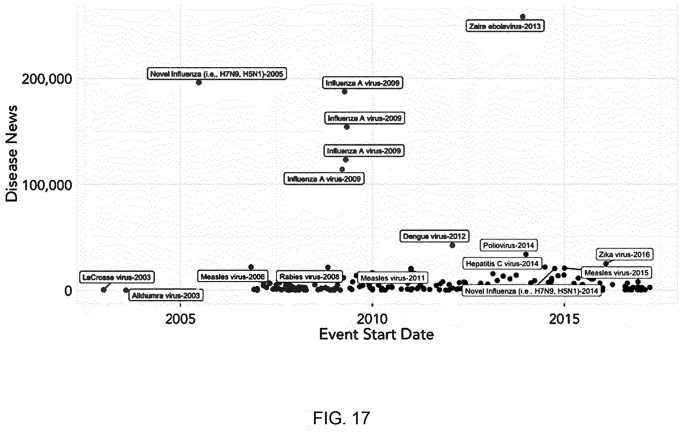

Figure 17 is a graph illustrating an embodiment of estimated media reporting. In the example shown, Figure 17 presents the estimated media reporting for each event, plotted against the year of outbreak occurrence; several notable events are outliers (*Zaire ebolavirus* 2013, pandemic influenza 2009), and there are generally higher levels of reporting for infectious disease events in United States. The impact of geography on outbreak reporting is not surprising, and is addressed in the primary empirical analysis.

The outcome variable measures the intensity of reporting on each sampled outbreak. This variable was constructed by extracting the sum total of media reports for the outbreak period, plus a "tail" period of roughly 2 months (60 days) that captures potentially lagged but significant reporting for small or isolated outbreaks. Alternative variable constructions, such as measuring "peak" reporting (maximum monthly reports during outbreak period + 60 days) were also developed and empirically tested, to ensure robustness to alternate estimation techniques. The search terms included the pathogen name, alternates and spellings, and known abbreviations and acronyms. Where acronyms were employed, other logical operators and terms were included to avoid biasing the results. For example, "MERS" was required to include "CoV", "virus", or other associated terms (e.g., "outbreak") to ensure that the resulting quantity reflected disease-related reporting, rather than unrelated acronyms and entities.

It is important to consider other sources of variation in media reporting, including the generally increasing level of media reporting over time, as well as potential surges (or troughs) in reporting during particular periods of time. For this reason, another variable was constructed, measuring the total volume of media reporting during the outbreak event and 60 day tail. This variable functions as a denominator, allowing us to model the *relative* proportion of media attention allocated to each pathogen.

The primarily empirical validation consists of a battery of negative binomial regressions. A negative binomial estimator is selected because the outcome variable of interest is a count (sum) of reports and is over-dispersed; ordinary least squares (OLS) regression models were estimated as robustness checks, with similar results.

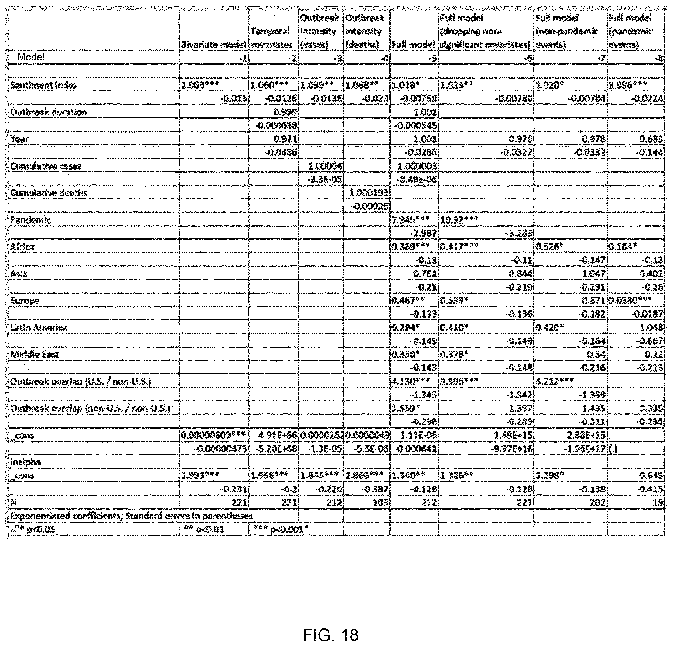

Figure 18 is a table illustrating an embodiment of regressions estimating correlates of media outbreak coverage. In the example shown, regressions move from simple bivariate models to models incorporating temporal, spatial, and event-level covariates. Regression coefficients are presented as incident-rate ratios (IRR), which have an intuitive interpretation. An IRR of 1.01 would indicate that for each one unit increase in sentiment score, reporting is estimated to increase by 1%; an IRR of 0.99 would indicate that a one unit increase in sentiment score would lead to a 1% decrease in reporting, all else being equal.

The denominator (total media reports) is included as an exposure/offset variableconceptually, it captures the number of reports which could have been about the outbreak (i.e., those that were "at risk").

Model 1 ("Bivariate model") is a simple bivariate analysis, and shows a positive, statistically-significant relationship between the Index and outbreak reporting. The effect is significant; each unit increase is associated with a 6 percent increase in reporting intensity. As Model 2 ("Temporal covariates") shows, this estimate is stable as temporal control, including outbreak duration and year of occurrence are taken into account. Models 3 ("Outbreak intensity (cases)") and 4 ("Outbreak intensity (deaths)") incorporate covariates measuring outbreak intensity, captured by cumulative cases and deaths, and the Index remains positively and significantly associated with increased reporting.

Model 5 ("Full model") includes a full vector of covariates. This includes variables measuring the geographic region in which the outbreak occurred, to account for systematic spatial variation in coverage- notably, less news coverage of events in Africa, Latin America, and Asia, relative to events in the United States and other high-income countries. Model 5 also includes a variable measuring outbreak scale, specifically whether the event is classified as a global pandemic, due to its spread across multiple continents. This model includes two variables which account for potential which could arise from the spatial expansion of an outbreak. An outbreak which begins in Country X but spreads to Country Y, and causes >=100 cases, could be included in the dataset as a discrete event. However, the outbreak periods for these events would overlap, leading to potential "double counting" of media reports. "Overlap US" is a binary variable which measures whether a particular event overlaps with a etiologically-related outbreak in the United States. "Overlap non-US" measures whether an event overlaps with another, etiologically-related outbreak occurring in another (non-U.S.) locality. Because cumulative death data is missing for approximately half of the outbreaks in the dataset, the cumulative case variable is used to measure outbreak intensity. In this model specification, the Index remains positive and statistically significant, though the coefficient estimate decreases to a roughly 2% estimated increase in reporting per each unit increase in the Index score. The coefficient estimate is stable when statistically insignificant covariates are dropped, in Model 6 ("Full model (dropping non-significant covariates)").

Lastly, Models 7 and 8 present estimates on specific sub-samples. Model 7 ("Full model (non-pandemic events)") estimates the relationship between the Index and reporting intensity during non-pandemic events; Model 8 ("Full model (pandemic events)") presents an estimate for the small number of pandemic events in the dataset (n=19). Strikingly, the Sentiment Index coefficient is substantially larger for pandemic events, with an estimated 10 percent increase in reporting per 1 unit increase in Index score, versus an estimated 2 percent increase in reporting for non-pandemic events-despite a small N and quite limited degrees of freedom. This suggests that pathogens with known pandemic potential and high-Index scores could have particularly severe impacts on public attitudes and behavior.

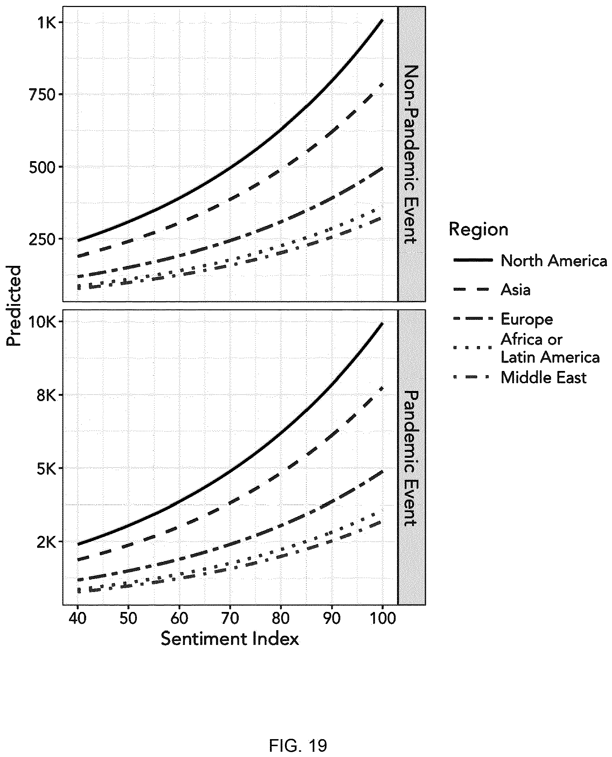

Figure 19 is a graph illustrating an embodiment of a relation between a sentiment index and predicted outbreak reporting intensity across pandemic and non-pandemic events. In the example shown, the predicted values are derived by using only significant variables in the full model (Model 6). The exposure/offset variable is fixed on the number of total news reports in the most recent month (July 2017) and is equal roughly to 2.1 million reports. Also, the model is fitted to the outbreaks that have neither U.S. nor non-U.S. reporting overlaps. In other words, the outbreak overlap variables are forced to be equal zero. This graphic also generates disaggregated estimates by geographic region, taking into account the varying levels of reporting likely to arise in U.S. media during outbreaks overseas. The slope is positive in both panels, with a substantial increase in estimated reporting from the bottom to the top of the Index scale; however the rate of increase is far steeper for pandemic events, with substantially wider marginal increases in reporting for outbreaks affecting the United States.

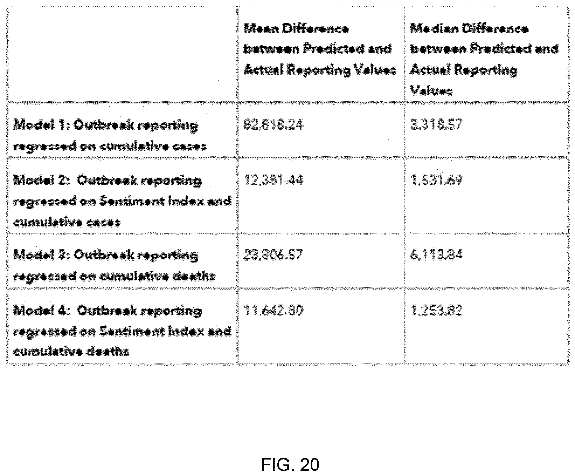

Figure 20 is a table illustrating an embodiment of a gap between predicted and actual reporting values comparing the sentiment index versus cumulative cases and deaths. In the example shown, media coverage is complex, and driven by a wide range of factors including linguistic differences, ease of media access and logistical capacity to report, relationships between pairs (and groups) of countries, and the perceived importance ("newsworthiness") of specific countries. Although the purpose of the backtesting and empirical analysis is not to build an ideal predictive model for media coverage, it is nonetheless highly informative to compare the predictive power of the Sentiment Index versus other epidemiologic variables such as cumulative cases and/or cumulative deaths.

The results present in the table of Figure 20 show that the Index values provide a significantly better prediction of media coverage intensity than epidemiologic indicators. The mean difference between predicted and actual media reports for Model 1 (bivariate, using cumulative cases as a predictor) is nearly 83,000, and the median difference is approximately 3,300. The mean difference for Model 2 (bivariate, using Sentiment Index and cumulative cases as a predictor) is approximately 12,000, and the median difference is approximately 1,500. Using a second epidemiologic trigger-cumulative deaths-yields similar results. The mean difference for Model 3 (cumulative deaths) is nearly 24,000, with a median difference of approximately 6,000; Model 4 (Sentiment Index and cumulative deaths) generates more accurate predictions: a mean difference of roughly 11,600 reports, and a median difference of roughly 1,250 reports.

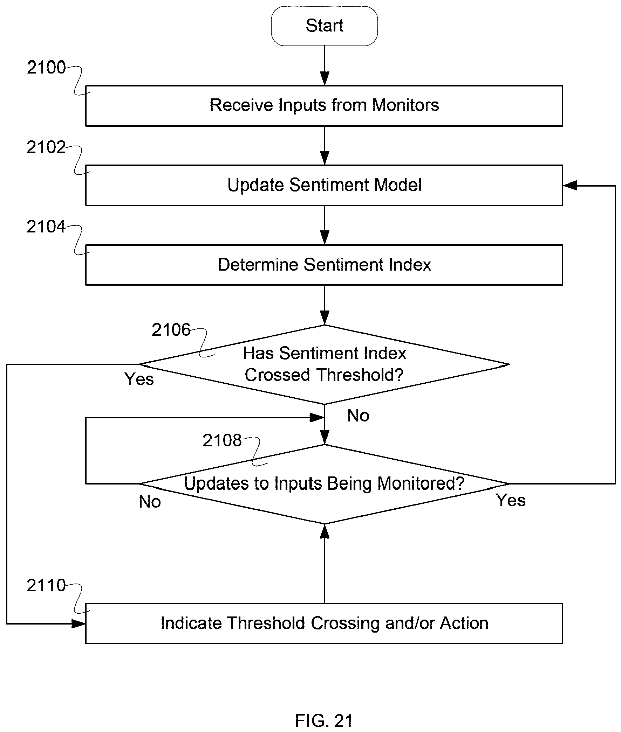

Figure 21 is a flow diagram illustrating an embodiment of a process for a sentiment index to be used to trigger an action when the sentiment index score crosses a threshold. In some embodiments, the process of Figure 21 is used to update the process of Figure 2 (e.g., the step 2102 executes again the entire process of Figure 2 or appropriate portions of it - for example, those that are changed). In the example shown, in 2100 inputs are received from monitors. For example, any inputs to the sub-indices upon which the sentiment index is based are monitored and, in the event that a factor changes, the sub-index based on the changed factor is updated and then the sentiment index as a whole is updated as well. In some cases, the monitors track the identification of new symptoms associated with a pathogen, changes to the case fatality ratio, changes to therapeutics, the development of vaccines, etc. In 2102, the sentiment model is updated. In 2104, the sentiment index is determined. In 2106, it is determined whether the sentiment index has crossed a threshold. In the event that the sentiment index has crossed a threshold, in 2110 it is indicated that a threshold has been crossed and/or indicate an action, and control passes to 2108. For example, in some cases the system can automatically indicate to an application system that an action needs to be taken. For instance, a system for determination of payment for insurance can be triggered (e.g., for a catastrophe bond, a business interruption policy, for weather coverage (e.g., hurricane policy), for risk policies, etc.). In the event that the sentiment index has not crossed a threshold, then in 2108 it is determined whether there are any updates to inputs being monitored. In the event that there are updates to inputs being monitored, control passes to 2102. In the event that there are not updates to inputs being monitored, control passes to 2108.

In some embodiments, the Sentiment Index is dynamic, with changes occurring in real-time based on regular (e.g., daily, weekly, or bi-weekly) updates of input data, such as available intervention measures, the case-fatality ratio, and other features of an outbreak. In reconstructed historical outbreaks of novel pathogens (e.g., SARS, Zika, etc.), using contemporary reporting from the time, it has been found that scores vary substantially as more becomes known about the attributes and behaviors of diseases. In some embodiments, news media is used for dynamically updating the Sentiment Index. For example, news articles and/or social media article are scraped about a particular outbreak, using customized search terms (e.g., pathogen name, description, characteristics, etc.); a fear score is assigned by identifying all of the disease symptoms listed in each article and scoring the symptoms by applying the morbidity catalog. Scores for all articles can be combined and normalize in a given time period (e.g., day, week, etc.) to produce an ongoing "ticker" for the level of fear represented within media reporting over time. In some embodiments, this media score is incorporated into the Index as a new sub-index: it is weighted and combined with the morbidity, mortality, transmission, therapeutics, vaccines, and novelty sub-indices in order to derive a new sentiment index.

Although the foregoing embodiments have been described in some detail for purposes of clarity of understanding, the invention is not limited to the details provided. There are many alternative ways of implementing the invention. The disclosed embodiments are illustrative and not restrictive.

## Claims

1. A system for a sentiment index, comprising:
an interface configured to:
receive a request to determine the sentiment index;
a processor configured to:
determine a morbidity sub-index;
determine a mortality sub-index; and
determine the sentiment index based at least in part on the morbidity sub-index and the mortality sub-index.

2. The system as in claim 1, wherein determining the morbidity sub-index comprises determining a fear score for a symptom or combination of symptoms.

3. The system as in claim 1, wherein determining the morbidity sub-index comprises determining a pathogen fingerprint of symptoms.

4. The system as in claim 3, wherein determining the morbidity sub-index comprises determining a pathogen fear score using the pathogen fingerprint of symptoms and a fear score for one or more symptoms in the pathogen fingerprint of symptoms.

5. The system as in claim 1, wherein determining the mortality sub-index comprises determining a case fatality ratio statistic using a decision rule-guided selection.

6. The system as in claim 5, wherein determining the mortality sub-index comprises determining a normalized case fatality ratio statistic from the case fatality ratio statistic.

7. The system as in claim 1, wherein the processor is further configured to determine a preventability sub-index, wherein the sentiment index is based at least in part the preventability sub-index.

8. The system as in claim 7, wherein the preventability sub-index comprises determining one of: whether a vaccine is widely available; and whether a vaccine is limitedly available.

9. The system as in claim 1, wherein the processor is further configured to determine a treatability sub-index, wherein the sentiment index is based at least in part the treatability sub-index.

10. The system as in claim 9, wherein the treatability sub-index comprises determining one of: whether a cure is available; whether countermeasures are available to reduce severity or duration; and whether post-exposure prophylaxis is available.

11. The system as in claim 1, wherein the processor is further configured to determine a transmission sub-index.

12. The system as in claim 11, wherein determining the transmission sub-index comprises determining a fear score associated with a transmission mechanism using a survey.

13. The system as in claim 12, wherein the survey comprises presenting a question to receive a fear rating.

14. The system as in claim 1, wherein the processor is further configured to determine a novelty sub-index, wherein the sentiment index is based at least in part the novelty sub-index.

15. The system as in claim 14, wherein determining the novelty sub-index comprises determining a fear score associated with a novelty using a survey.

16. The system as in claim 15, wherein the survey comprises presenting a question to receive a fear rating.

17. The system as in claim 1, wherein determining a sentiment index includes determining a weighted summing of one or more sub-indexes.

18. A method for a sentiment index, comprising:
receiving a request to determine the sentiment index;
determining, using a processor, a morbidity sub-index;
determining a mortality sub-index; and
determining the sentiment index based at least in part on the morbidity sub-index and the mortality sub-index.

19. A computer program product for a sentiment index, the computer program product being embodied in a non-transitory computer readable storage medium and comprising computer instructions for:
receiving a request to determine the sentiment index;
determining, using a processor, a morbidity sub-index;
determining a mortality sub-index; and
determining the sentiment index based at least in part on the morbidity sub-index and the mortality sub-index.
